# EUROPEAN PATENT SPECIFICATION

(11) **EP 4 656 233 B1**
(45) Date of publication and mention of the grant of the patent: **14.10.2026**
(21) Application number: 25178338.7
(22) Date of filing: 22.05.2025
(51) Int. Cl.: A61N 1/05, A61N 1/372, A61N 1/375

(54) **BIOSTIMULATOR TRANSPORT SYSTEM HAVING LOCATION GUIDES**
BIOSTIMULATORTRANSPORTSYSTEM MIT POSITIONSFÜHRUNGEN
SYSTÈME DE TRANSPORT DE BIOSTIMULATEUR AYANT DES GUIDES DE LOCALISATION

(30) Priority: 24.05.2024 US 202463651909 P; 21.05.2025 US 202519215129
(43) Date of publication of application: 03.12.2025
(73) Proprietor: Pacesetter, Inc., Sylmar, CA 91342 (US)
(72) Inventor: ARNAR, Bernhard, Sylmar, CA 91342 (US); JACKSON, Aaron, Sylmar, CA 91342 (US); KRANS, Mark, Sylmar, CA 91342 (US)
(74) Representative: Barker Brettell Sweden AB

(56) References cited:
- US-A1- 2021 068 868
- US-B2- 9 095 700

## Description

### TECHNICAL FIELD

The present disclosure relates to biostimulators and related biostimulator systems. More specifically, the present disclosure relates to leadless biostimulators and related systems useful for septal pacing.

### BACKGROUND

Cardiac pacing by an artificial pacemaker provides an electrical stimulation of the heart when its own natural pacemaker and/or conduction system fails to provide synchronized atrial and ventricular contractions at rates and intervals sufficient for a patient's health. Such antibradycardial pacing provides relief from symptoms and even life support for hundreds of thousands of patients. Cardiac pacing may also provide electrical overdrive stimulation to suppress or convert tachyarrhythmias, again supplying relief from symptoms and preventing or terminating arrhythmias that could lead to sudden cardiac death.

Leadless cardiac pacemakers incorporate electronic circuitry at the pacing site and eliminate leads, thereby avoiding shortcomings associated with conventional cardiac pacing systems. Leadless cardiac pacemakers can be anchored at the pacing site, e.g., in a right ventricle and, for dual-chamber pacing, in a right atrium, by an anchor. A delivery system can be used to deliver the leadless cardiac pacemakers to the target anatomy.

Cardiac pacing of the His-bundle is clinically effective and advantageous by providing a narrow QRS affecting synchronous contraction of the ventricles. His-bundle pacing in or near a membranous septum of a heart, however, has some drawbacks. The procedure is often long in duration and requires significant fluoroscopic exposure. Furthermore, successful His-bundle pacing cannot always be achieved. Pacing thresholds are often high, sensing is challenging, and success rates can be low.

Pacing at the left bundle branch area (LBBAP) is an alternative to His-bundle pacing. LBBAP involves pacing past the His-bundle toward the right ventricular apex. More particularly, a pacing site for LBBAP pacing is typically below the His-bundle, on the interventricular septal wall. LBBAP can prevent pacing induced cardiomyopathy in a bradycardia patient population. LBBAP can also be an effective alternative to cardiac resynchronization therapy in treating heart failure patients. To achieve optimal results, the pacing site for physiological LBBAP can be high on the interventricular septal wall, in the region close to the tricuspid valve and pulmonary artery outflow track. Furthermore, the pacing site may be at a depth of up to 1.5 cm within the septal wall.

US 9095700 discloses a therapy assembly configured for at least partial insertion in a living body. A plurality of fixation structures are disposed radially around the therapy delivery element proximate the electrodes. The fixation structures include wires having a diameter in a range between about 0.004 inches and about 0.020 inches. The wires have a first end attached to the therapy delivery element and a second end attached to a sliding member configured to slide along the therapy delivery element. The fixation structures are configured to collapse inward to a collapsed configuration when inserted into a lumen of an introducer and to deploy to a deployed configuration when the introducer is retracted. A fitting is located at proximal end of the introducer that releasably locks the therapy delivery element to the introducer, such that torque applied to the fitting is substantially transmitted to the distal end of the therapy assembly.

US 2021/068868 discloses an implantation and/or retrieval device for a leadless cardiac pacing device that includes a first elongate shaft including a lumen; a second elongate shaft slidably disposed within the lumen of the first elongate shaft; an end cap assembly fixedly attached to a distal end of the first elongate shaft; and a plurality of wires attached to the second elongate shaft and extending distally from the end cap assembly, the plurality of wires being movable relative to the end cap assembly. The plurality of wires is configured to engage a proximal hub of the leadless cardiac pacing device. The plurality of wires forms a plurality of wire loops extending distally from the end cap assembly.

### SUMMARY

Significant challenges are associated with delivering a leadless cardiac pacemaker to the left bundle branch area pacing (LBBAP) pacing site. For example, the leadless cardiac pacemaker may be required to be delivered through limited available space within the right ventricle to engage a particular location at a particular angle and extend deep, e.g., 1 cm, into the septal wall. Several attempts are often required to achieve such septal wall engagement because existing leadless pacemakers may not fit, or may interfere with heart structures, when placed at the optimal pacing site for LBBAP. Furthermore, a shape of the septal wall can bias the leadless cardiac pacemaker into anatomical structures, e.g., posterior and anterior ventricular grooves, which are not at the target location. Thus, there is a need for a leadless biostimulator transport system that can deliver a leadless cardiac pacemaker to engage the interventricular septal wall at a target location and/or angle such that the biostimulator can penetrate deep into the septal wall to pace the LBB directly for improved conduction system capture and battery life.

The invention is defined in the appended claims. Aspects, embodiments and examples disclosed herein which do not fall within the scope of the appended claims do not form part of the invention, and are merely provided for illustrative purposes.

A biostimulator transport system is described. In an embodiment, the biostimulator transport system includes a biostimulator coupling along a central axis. The biostimulator coupling is configured to hold a biostimulator. The biostimulator transport system includes one or more location guides deployable radially outward from the central axis to engage anatomical landmarks. The biostimulator coupling is advanceable along the central axis relative to the one or more location guides when the one or more location guides are in a deployed state.

A biostimulator system is described. In an embodiment, the biostimulator system includes a biostimulator mounted on the biostimulator transport system. A method of delivering the biostimulator to a target anatomy using the biostimulator transport system is also described.

The above summary does not include an exhaustive list of all aspects of the present invention. It is contemplated that the invention includes all systems and methods that can be practiced from all suitable combinations of the various aspects summarized above, as well as those disclosed in the Detailed Description below and particularly pointed out in the claims filed with the application. Such combinations have particular advantages not specifically recited in the above summary.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings.
FIG. 1 is a diagrammatic cross section of a patient heart illustrating an example implantation of a biostimulator in a target anatomy, in accordance with an embodiment.
FIG. 2 is a perspective view of a biostimulator system, in accordance with an embodiment.
FIG. 3 is a perspective view of a biostimulator system in an undeployed state, in accordance with an embodiment.
FIG. 4 is a perspective view of a biostimulator system in a deployed state, in accordance with an embodiment.
FIG. 5 is a top view of a biostimulator system in a deployed state, in accordance with an embodiment.
FIG. 6 is a side view of a biostimulator system in a deployed state, in accordance with an embodiment.
FIG. 7 is a perspective view of a biostimulator system deployed in a target anatomy, in accordance with an embodiment.
FIG. 8 is a perspective view of a biostimulator system in an undeployed state, in accordance with an embodiment.
FIG. 9 is a perspective view of a biostimulator system in a deployed state, in accordance with an embodiment.
FIG. 10 is a perspective view of a biostimulator system in a deployed state, in accordance with an embodiment.
FIG. 11 is a perspective view of a biostimulator system deployed in a target anatomy, in accordance with an embodiment.
FIG. 12 is a side view of a biostimulator system in an undeployed state, in accordance with an embodiment.
FIG. 13 is a perspective view of a biostimulator system in an undeployed state, in accordance with an embodiment.
FIG. 14 is a side view of a biostimulator system in a deployed state, in accordance with an embodiment.
FIG. 15 is a perspective view of a biostimulator system in a deployed state, in accordance with an embodiment.
FIG. 16 is a perspective view of a biostimulator system deployed in a target anatomy, in accordance with an embodiment.
FIG. 17 is a side view of a biostimulator system in an undeployed state, in accordance with an embodiment.
FIG. 18 is a side view of a biostimulator system in a deployed state, in accordance with an embodiment.
FIG. 19 is a perspective view of a biostimulator system deployed in a target anatomy, in accordance with an embodiment.
FIG. 20 is a side view of a biostimulator system in an undeployed state, in accordance with an embodiment.
FIG. 21 is a side view of a biostimulator system in a deployed state, in accordance with an embodiment.
FIG. 22 is a perspective view of a biostimulator system deployed in a target anatomy, in accordance with an embodiment.
FIG. 23 is a perspective view of a biostimulator system in an undeployed state, in accordance with an embodiment.
FIG. 24 is a side view of a biostimulator system in an undeployed state, in accordance with an embodiment.
FIG. 25 is a side view of a biostimulator system in a deployed state, in accordance with an embodiment.
FIG. 26 is a top view of a biostimulator system in an undeployed state, in accordance with an embodiment.
FIG. 27 is a top view of a biostimulator system in a deployed state, in accordance with an embodiment.
FIG. 28 is a flowchart of a method of implanting a biostimulator in a target tissue.

### DETAILED DESCRIPTION

Embodiments describe a biostimulator transport system for delivering a biostimulator to perform pacing, e.g., septal pacing. The biostimulator may, however, be used in other applications, such as deep brain stimulation. Thus, reference to the biostimulator as being a cardiac pacemaker for pacing, or septal pacing, is not limiting.

In various embodiments, description is made with reference to the figures. However, certain embodiments may be practiced without one or more of these specific details, or in combination with other known methods and configurations. In the following description, numerous specific details are set forth, such as specific configurations, dimensions, and processes, in order to provide a thorough understanding of the embodiments. In other instances, well-known processes and manufacturing techniques have not been described in particular detail in order to not unnecessarily obscure the description. Reference throughout this specification to "one embodiment," "an embodiment," or the like, means that a particular feature, structure, configuration, or characteristic described is included in at least one embodiment. Thus, the appearance of the phrase "one embodiment," "an embodiment," or the like, in various places throughout this specification are not necessarily referring to the same embodiment. Furthermore, the particular features, structures, configurations, or characteristics may be combined in any suitable manner in one or more embodiments.

The use of relative terms throughout the description may denote a relative position or direction. For example, "distal" may indicate a first direction along a longitudinal axis of a biostimulator. Similarly, "proximal" may indicate a second direction opposite to the first direction. Such terms are provided to establish relative frames of reference, however, and are not intended to limit the use or orientation of a biostimulator, a biostimulator transport system, or a biostimulator system to a specific configuration described in the various embodiments below.

In an aspect, a biostimulator transport system includes location guides that can be deployed to engage anatomical landmarks. The biostimulator transport system can utilize the anatomical landmarks to guide a biostimulator to a target location. More particularly, the biostimulator transport system can include a biostimulator coupling to hold the biostimulator, and when the location guides interact with anatomical structures, a position of a target location relative to the anatomical landmarks can be known or determined. The biostimulator may therefore be directed to and advanced toward target tissue at the target location. For example, the location guides can engage the posterior and anterior grooves 152a, 152b of the ventricle, which may be used as landmarks to direct the biostimulator 100 toward the septal wall.

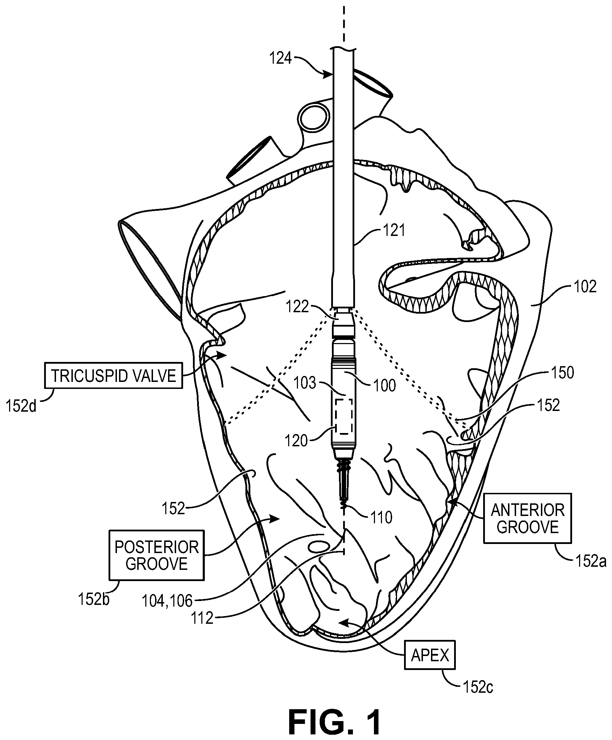

Referring to FIG. 1, a diagrammatic cross-section of a patient heart illustrating an example implantation of a biostimulator in a target anatomy is shown in accordance with an embodiment. The diagram shows a biostimulator 100 attached to a patient heart 102 with a body 103 of the biostimulator 100 positioned toward a ventricular apex 152c. A leadless biostimulator system 124, e.g., a cardiac pacing system, can include the biostimulator 100. The biostimulator 100 can be implanted in the patient heart 102, and can be leadless (and thus, may be a leadless cardiac pacemaker). The biostimulator 100 can be placed in a cardiac chamber, such as a right atrium and/or right ventricle of the heart 102, or attached to an inside or outside of the cardiac chamber. For example, the biostimulator 100 can be attached to an interventricular septal wall 104 of the heart 102. More particularly, the biostimulator 100 can be delivered to an interventricular septum, and one or more elements, such as a fixation element 110, can pierce the interventricular septal wall 104 of the septum to engage and anchor the biostimulator 100 to a target tissue 106.

In an embodiment, the biostimulator 100 can extend along a central axis 112. For example, a biostimulator transport system 121 can include the central axis 112, e.g., defined by an elongated catheter through which the central axis 112 extends, and the biostimulator 100 can be coupled to the biostimulator transport system 121 with a body 103 of the biostimulator 100 extending in line with the central axis 112. The biostimulator transport system 121 can include a biostimulator coupling 122 along the central axis 112, and the biostimulator 100 can be mounted on the biostimulator coupling 122. The combined biostimulator transport system 121 and biostimulator 100 can constitute a biostimulator system 124 used to electrically interact with the target tissue 106. More particularly, the body 103 of the biostimulator 100 can contain an electronics compartment 120 containing circuitry configured to deliver and/or receive electrical signals from the target tissue 106, e.g., to pace or sense the target tissue 106. The circuitry may, for example, generate pacing impulses that are delivered into the target tissue 106 through the fixation element 110 or another electrode of the biostimulator 100.

When the biostimulator 100 is delivered to and plunged into the septum of the heart 102, the fixation element 110 may be positioned for deep septal pacing at a target anatomy, such as at a bundle branch in the septum. For example, an electrode of the biostimulator 100, which may include the fixation element 110, can be positioned at a left bundle branch in the septum. The biostimulator 100 may deliver pacing impulses through the pacing electrode to the target anatomy. Accordingly, the pacing electrode can be located to effectively probe and pace the target anatomy, while the body 103 can be placed in a safe and non-obstructive location within the heart chamber.

The biostimulator 100 can be a leadless cardiac pacemaker that can perform cardiac pacing and that has many of the advantages of conventional cardiac pacemakers while extending performance, functionality, and operating characteristics. In a particular embodiment, the biostimulator 100 can use two or more electrodes located on or within the body 103 of the biostimulator 100 for pacing the cardiac chamber upon receiving a triggering signal from at least one other device within the patient anatomy. The two or more electrodes of the biostimulator 100 can include an electrode of the fixation element 110 that acts as an active electrode. The electrodes can deliver pacing pulses to target anatomies, such as bundle branches within the septum of the heart 102, to perform pacing, e.g., deep septal pacing, and optionally, can sense electrical activity from the muscle. The electrodes may also communicate bidirectionally with at least one other device within or outside the patient anatomy.

The body 103 can contain a primary battery to provide power for pacing, sensing, and communication, which may include, for example, bidirectional communication. The body 103 can optionally have the electronics compartment 120 (shown by hidden lines) to hold circuitry adapted for different functionality. For example, the electronics compartment 120 can contain pacing circuitry for sensing cardiac activity from the electrodes, for receiving information from at least one other device via the electrodes, for generating pacing pulses for delivery to the target tissue 106 via the electrode(s), or other circuitry. Accordingly, the pacing circuitry can be electrically connected to the electrode(s). The electronics compartment 120 may contain circuits for transmitting information to at least one other device via the electrodes and can optionally contain circuits for monitoring device health. The circuitry of the biostimulator 100 can control these operations in a predetermined manner. In some implementations of a cardiac pacing system, cardiac pacing is provided without a pulse generator located in the pectoral region or abdomen, without an electrode-lead separate from the pulse generator, without a communication coil or antenna, and without an additional requirement of battery power for transmitted communication.

Continuing to refer to FIG. 1, the fixation element 110 can retain the body 103 within a target tissue 106. For example, the fixation element 110 can include a helix, e.g., a helically wound wire having a sharpened tip, to pierce and screw into the target tissue 106. The target tissue 106 may be a predetermined portion of the target anatomy, such as a left bundle branch in the septal wall.

In an embodiment, the biostimulator 100 includes an attachment feature to allow a biostimulator transport system 121 to grasp and retain the biostimulator 100 *in vivo.* The attachment feature may be mounted on a proximal body end of the body 103. The attachment feature may have a graspable shape, such as a button, a hook, a knob, or another shape. The attachment feature can be engaged by the biostimulator transport system 121, e.g., by the biostimulator coupling 122 or another component, to hold the body 103 relative to the biostimulator transport system 121.

As described below, the biostimulator transport system 121 can include one or more location guides 150. For example, the biostimulator transport system 121 can include several location guides 150. The location guides 150 may be deployable radially outward from the central axis 112. The location guides 150 are illustrated as dotted lines extending in different radial directions, relative to the central axis 112, from a catheter shaft of the biostimulator transport system 121. The dotted lines provide a representation of the location guides 150, however, the location guides 150 may have various structural embodiments, as described below.

When the location guides 150 deploy radially outward, the location guides 150 can engage anatomical landmarks 152 within the patient anatomy. The anatomical landmarks 152 can include various anatomical structures that can be used as a point of orientation in locating a target site for biostimulator implantation. More particularly, the target site can include the target tissue 106 having a predetermined location relative to the anatomical landmarks 152. For example, the anatomical landmarks 152 can include one or more of an anterior groove 152a, a posterior groove 152b, an apex 152c of the right ventricle, or a tricuspid valve 152d and the target tissue 106 can be on the septal wall above and/or between the anatomical landmarks 152. Accordingly, when the location guides 150 engage the anatomical landmarks 152, the location of the target tissue 106 can be determined and targeted by advancing the biostimulator 100 to the target tissue 106 for implantation.

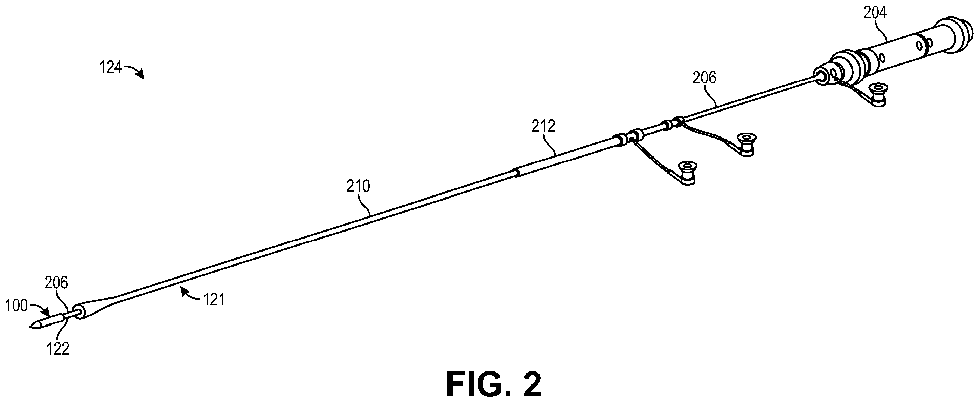

Referring to FIG. 2, a perspective view of a biostimulator system is shown in accordance with an embodiment. Leadless pacemakers or other leadless biostimulators can be delivered to or retrieved from a patient using delivery or retrieval systems. The leadless biostimulator system 124 can include delivery or retrieval systems, which may be catheter-based systems used to carry a leadless biostimulator 100 intravenously to or from a patient anatomy. The delivery or retrieval systems may be referred to collectively as transport systems, or biostimulator transport systems.

The biostimulator system 124 can include a biostimulator transport system 121. The biostimulator 100 can be attached, connected to, or otherwise mounted on the biostimulator transport system 121. For example, the biostimulator 100 can be mounted on an output shaft of the biostimulator transport system 121, as described below. The biostimulator 100 is thereby advanced intravenously into or out of the heart 102.

The biostimulator transport system 121 can include a handle 204 to control movement and operations of the biostimulator transport system 121 from outside of a patient anatomy. One or more elongated members can extend distally from the handle 204. For example, an inner sheath 206 can extend distally from the handle 204. The inner sheath 206 can extend to the biostimulator coupling 122 at a distal end of the biostimulator transport system 121.

The biostimulator transport system 121 can include an outer sheath 210. The outer sheath 210 can cover the biostimulator 100 and/or inner sheath 206 during delivery and implantation. The outer sheath 210 can extend over, and be longitudinally movable relative to, the inner sheath 206. The biostimulator transport system 121 may also include an introducer sheath 212 that can extend over, and be longitudinally movable relative to, the outer sheath 210. The introducer sheath 212 can cover a distal end of the outer sheath 210, the inner sheath 206, and the biostimulator 100 as those components are passed through an access device into the patient anatomy.

Several components of the biostimulator transport system 121 are described above by way of example. It will be appreciated, however, that the biostimulator transport system 121 may be configured to include additional or alternate components. More particularly, the biostimulator transport system 121 may be configured to deliver and/or retrieve the biostimulator 100 to or from the target tissue 106. Delivery and/or retrieval of the biostimulator 100 can include retaining the biostimulator 100 during transport to the target anatomy and rotation of the biostimulator 100 during implantation of the biostimulator 100 at the target tissue 106. Accordingly, the biostimulator transport system 121 can incorporate features to retain and rotate the biostimulator 100.

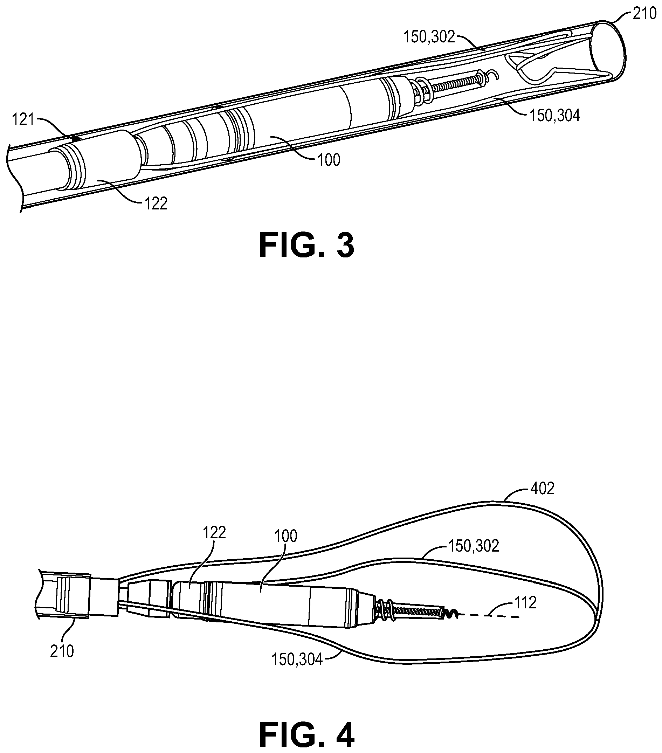

Referring to FIG. 3, a perspective view of a biostimulator system in an undeployed state is shown in accordance with an embodiment. In an undeployed state, the biostimulator transport system 121, which holds the biostimulator 100 by the biostimulator coupling 122, can be retracted into the outer sheath 210. For example, the outer sheath 210 can be a protective sleeve, and the biostimulator transport system 121 can be positioned within the outer sheath 210 such that the biostimulator 100 is held in the protective sleeve. One or more location guides 150 can be positioned within the outer sheath 210 around the biostimulator 100. For example, the location guides 150 can include a first location guide 302 and a second location guide 304 extending distally from the biostimulator coupling 122 alongside the biostimulator 100.

In the undeployed state, the location guides 150 can be folded to a low profile configuration. More particularly, the location guides 150 can fit within the outer sheath210. When folded into the outer sheath 210 and placed along the biostimulator 100, the first location guide 302 may be diametrically opposed to the second location guide 304 at opposite points along an inner diameter of the outer sheath 210.

Referring to FIG. 4, a perspective view of a biostimulator system in a deployed state is shown in accordance with an embodiment. The location guides 150 can be formed from wires. For example, the wires may be shape memory alloy wires having a predefined shape in an unconstrained configuration when the biostimulator transport system 121 is in the deployed state and when the location guides 150 are unconstrained. More particularly, when the outer sheath 210 is retracted relative to the biostimulator 100, the biostimulator transport system 121 can transition from the undeployed state to the deployed state and the location guides 150 can expand from the low profile configuration to the unconstrained configuration. In some embodiments, the location guides 150 may automatically expand from the low profile configuration to the unconstrained configuration when the biostimulator transport system 121 is in the deployed state.

The location guides 150 can be fully deployed when a distal end of the outer sheath 210 is proximal to the biostimulator coupling 122. In the deployed state, the location guides 150 can expand to the unconstrained configuration with the first location guide 302 can be diametrically opposed to the second location guide 304. For example, the first location guide 302 can extend radially outward from the central axis 112 on a first side of the biostimulator 100 and the second location guide 304 can extend radially outward from the central axis 112 on a second side of the biostimulator 100. Accordingly, the first location guide 302 can engage an anatomical structure on the first side of the biostimulator 100 and the second location guide 304 can engage an anatomical structure on the second side of the biostimulator 100.

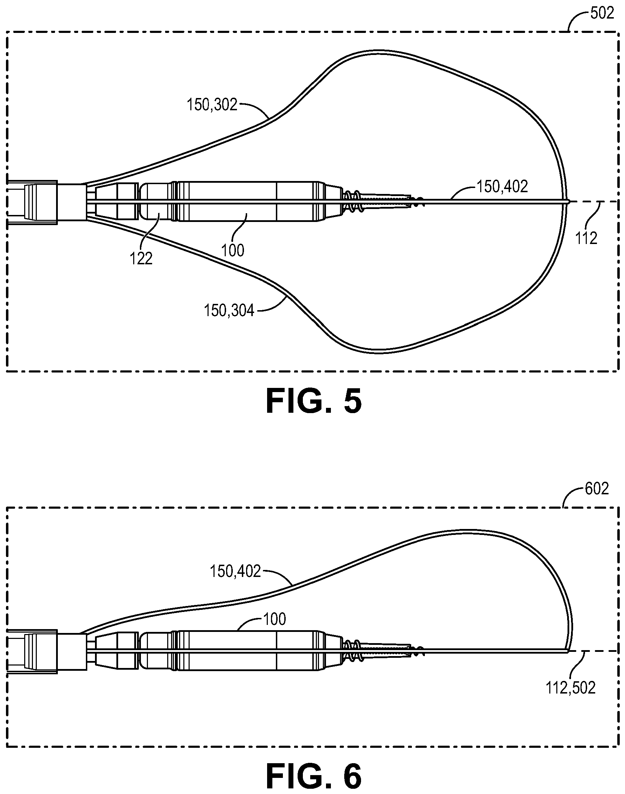

The location guides 150 can include an additional location guide extending in a different direction than the first location guide 302 and the second location guide 304. For example, location guides 150 can include a rear location guide 402 extending radially outward from the central axis 112. Whereas the first location guide 302 and the second location guide 304 can extend radially outward within a lateral plane 502 (FIG. 5) the rear location guide 402 can extend radially outward along a rear plane 602 (FIG. 6). The rear plane 602 can be orthogonal to the lateral plane 502.

Referring to FIG. 5, a top view of a biostimulator system in a deployed state is shown in accordance with an embodiment. When the first location guide 302 and the second location guide 304 deploy from the undeployed state to the deployed state, the wires can bow radially outward. More particularly, at least a portion of the location guides 150 that extend longitudinally parallel to the central axis 112 in the undeployed state can extend radially outward from the central axis 112 in the deployed state. For example, the first location guide 302 can extend from a proximal end proximal to the biostimulator coupling 122 to a distal end where the first location guide 302 intersects the second location guide 304 and the rear location guide 402.

The pre-shaped wire of the location guides 150 can expand and fit into anatomical landmarks 152 within the right ventricle. For example, the first location guide 302 can engage an anterior groove 152a of the ventricle, and the second location guide 304 can engage a posterior groove 152b of the ventricle. Alternatively, location guides 150 may engage in an apex 152c or a free wall of the ventricle.

Referring to FIG. 6, a side view of a biostimulator system in a deployed state is shown in accordance with an embodiment. When the location guides 150 engage the grooves of the ventricle, the lateral plane 502 can be in line with the grooves. Accordingly, a rear plane 602 may extend orthogonal to the plane containing the grooves, e.g., lateral plane 502. As shown, the rear location guide 402 can extend radially outward along the rear plane 602 orthogonal to the lateral plane 502. Like the first location guide 302 and the second location guide 304, the rear location guide 402 can bow outward.

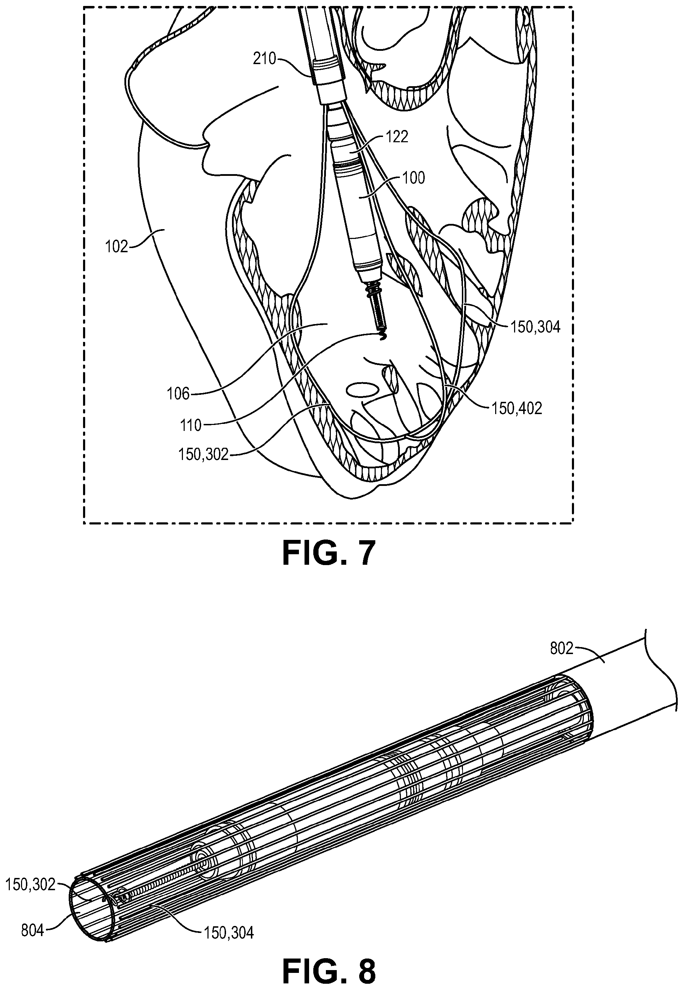

Referring to FIG. 7, a perspective view of a biostimulator system deployed in a target anatomy is shown in accordance with an embodiment. The bowed shape of the location guides 150 can conform to the ventricular wall. More particularly, a curvature of the rear location guide 402 can conform to a curvature of the ventricular wall. Accordingly, when the location guides 150 are expanded, the location guide 150 structure can conform to the heart 102 and support 2306 the biostimulator 100 within the ventricle.

The biostimulator 100 can be supported centrally within the ventricle. For example, the first and second location guides 302, 304 can engage the ventricular grooves and the rear location guide 402 can engage a free wall of the ventricle. Biostimulator 100 may be pointed downward toward the apex 152c of the heart 102. With the anatomical landmarks 152 engaged, the biostimulator 100 may be steered away from the rear location guide 402 to direct a fixation element 110 and/or electrode of the biostimulator 100 toward the target tissue 106 at a target location.

Steering of the biostimulator 100 may be augmented by a radiopaque material integrated in the location guides 150. For example, the wires may be radiopaque such that, when viewed under fluoroscopy, the wires give a picture of where the delivery catheter is in the ventricle. The location guides 150 can therefore provide visual feedback and structural support to physically center the delivery catheter and/or biostimulator 100 within the ventricle by pushing against the anatomical landmarks 152. The expanded structure occupies space within the ventricle and engages landmarks to aim the biostimulator 100 toward the target tissue 106.

When the biostimulator 100 is directed into the target tissue 106, the biostimulator transport system 121 can detach from the biostimulator 100. More particularly, the biostimulator coupling 122 can be actuated to release the biostimulator 100. The location guides 150 and biostimulator coupling 122 can be retracted into the outer sleeve 210. The biostimulator transport system 121 can be removed from the patient anatomy while the biostimulator 100 is left in the ventricle to pace the target tissue 106.

Referring to FIG. 8, a perspective view of a biostimulator system in an undeployed state is shown in accordance with an embodiment. Expansion of the location guides 150 may be controlled by relative movement between components of the biostimulator transport system 121. In an embodiment, the biostimulator transport system 121 includes an outer shaft 802 and an inner shaft 804. The shafts may be coaxially oriented, tubular structures. For example, the outer shaft 802 can include a first tubular shaft that slides over the inner shaft 804 in a distal direction and a proximal direction. Movement of the outer shaft 802 relative to the inner shaft 804 can increase or decrease a distance between the distal ends of the shafts.

In an embodiment, location guides 150 of the biostimulator transport system 121 extend between the outer shaft 802 and the inner shaft 804. More particularly, a proximal end of a location guide 150 can attach to the distal end of the outer shaft 802, and a distal end of the location guide 150 can attach to the distal end of the inner shaft 804. In the undeployed state, wires of the location guides 150 can lay flat against the inner shaft 804. For example, the location guides 150 can extend longitudinally, parallel to the central axis 112, and along an outer surface of the inner shaft 804. Accordingly, in the undeployed state, the location guides 150 can have a low profile to allow the biostimulator transport system 121 to access the right ventricle of the heart 102.

As described above, at least two wires of the location guides 150 can be diametrically opposed to each other. For example, the first location guide 302 can be a wire on an opposite side of the inner shaft 804 relative to the second location guide 304. The opposing location guides 150 may therefore be within the lateral plane 502, which can be oriented relative to the anatomical landmarks 152, such as the anterior and posterior grooves 152a, 152b of the ventricle.

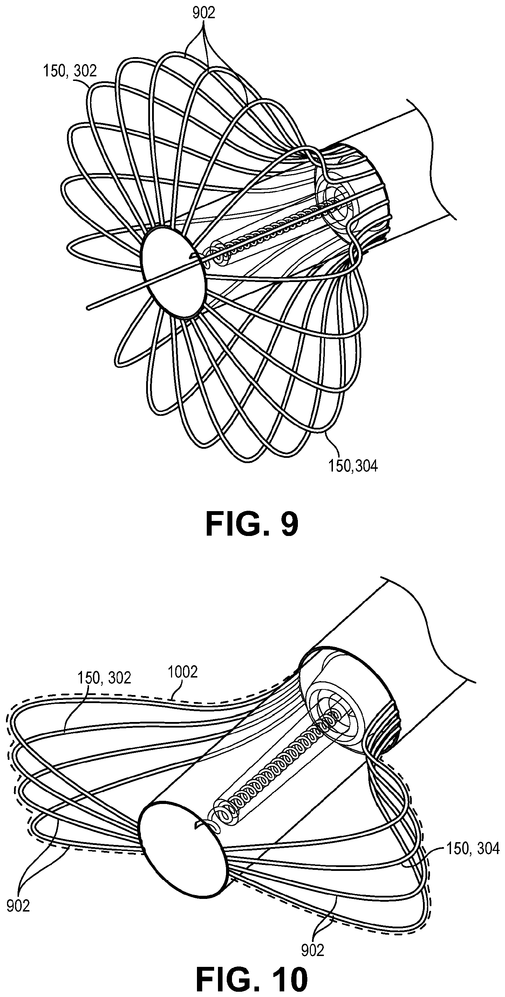

Referring to FIG. 9, a perspective view of a biostimulator system in a deployed state is shown in accordance with an embodiment. The location guides 150, which include the wires 902 extending longitudinally around the outer surface of the inner shaft 804, may be deployable from the undeployed state to the deployed state. For example, when the biostimulator transport system 121 is inside the ventricle, the outer shaft 802 can be translated forward over the inner shaft 804 to reduce the distance between the distal and proximal ends of the location guides 150. When the distance is reduced, the location guides 150 can deform, e.g., expand outward, within the ventricle. In the deployed state, the wires 902 can bow radially outward and, depending on the configuration of the wires 902, can use the structures of the ventricle to position the biostimulator transport system 121 and the biostimulator 100 for optimal location to implant the biostimulator 100.

The anatomical landmarks 152 can include the posterior and anterior grooves 152a, 152b, or the free wall, of the ventricle. The bowed wires 902 may extend uniformly from the central axis 112. For example, the wires 902 can be evenly distributed about the central axis 112.

Referring to FIG. 10, a perspective view of a biostimulator system in a deployed state is shown in accordance with an embodiment. Rather than being distributed evenly about the central access, the location guides 150 may expand in an asymmetrical and unbalanced manner. More particularly, one or more location guides 150 can extend in a first direction from the inner shaft 804 and one or more location guides 150 can extend in a second direction, opposite to the first direction, from the inner shaft 804. The diametrically opposed guides may orient toward respective grooves of the ventricle. More particularly, the first group of wires can extend toward the anterior groove 152a of the ventricle, and the second group of wires can extend toward the posterior groove 152b of the ventricle.

In an embodiment, the biostimulator transport system 121 includes a skin 1002 covering the wires 902 of the location guides 150. For example, the skin 1002 can be a webbing that covers the wires 902 to prevent snagging or pinching of tissue when the location guides 150 are expanded. By way of example, the skin 1002 may be formed from a tubular, woven material that covers the wires 902 in a low profile shape in the undeployed state, and deforms radially outward when the wires 902 are deployed to the deployed state.

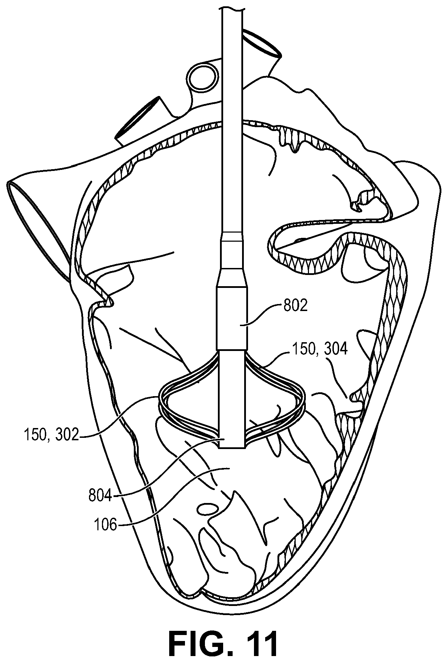

Referring to FIG. 11, a perspective view of a biostimulator system deployed in a target anatomy is shown in accordance with an embodiment. The location guides 150 may expand into contact with the ventricular wall to center the catheter of the biostimulator transport system 121 within the ventricle. More particularly, the location guides 150 can engage sidewalls of the ventricle to center the sheets above the apex 152c. The biostimulator 100 may be advanced from the inner sheath 206 toward the target tissue 106. The catheter component holding the biostimulator 100 can be pushed forward to expose the biostimulator 100 and to implant the biostimulator 100 into the target tissue 106. The biostimulator 100 can be implanted at the target tissue 106, which may have a predetermined relative position relative to the anatomical landmarks 152 that are engaged by the location guides 150. The outer shaft 802 can be retracted relative to the inner shaft 804 to stretch the wires 902 into the low profile, undeployed state. When the wires 902 lay flat against the inner sheath 206, the biostimulator transport system 121 may be removed from the patient anatomy, and the biostimulator 100 can be left in place to perform pacing.

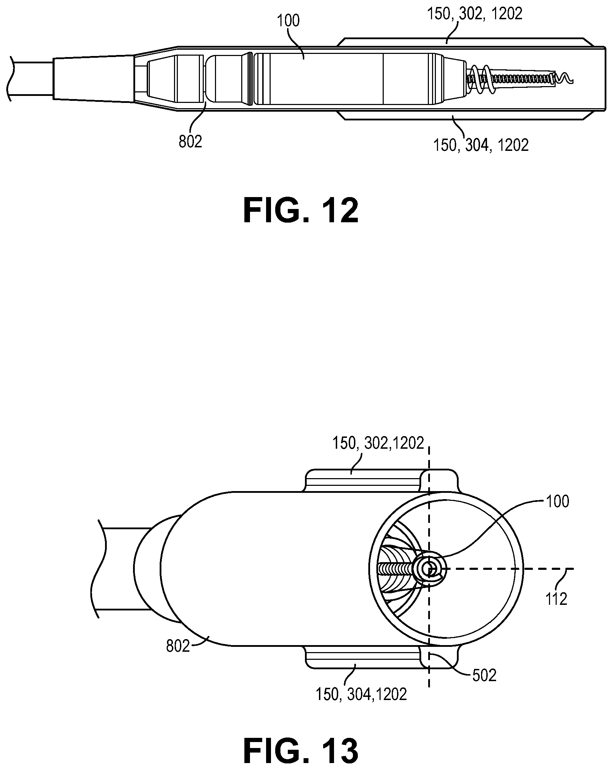

Referring to FIG. 12, a side view of a biostimulator system in an undeployed state is shown in accordance with an embodiment. The biostimulator transport system 121 can include inflatable location guides 150. For example, several location guides 150 can include inflatable elements 1202 disposed on an outer surface of the outer sheath 210. The inflatable elements 1202 may be deployable from the undeployed state to a deployed state.

In the undeployed state, the inflatable elements 1202 are deflated. Inflatable elements 1202 may extend longitudinally along the outer surface of the outer sheath 210. The biostimulator 100 can be retracted or stored within the outer sheath 210, radially inward from the inflatable elements 1202. In the deflated configuration, the inflatable elements 1202 may have a low profile.

Referring to FIG. 13, a perspective view of a biostimulator system in an undeployed state is shown in accordance with an embodiment. The inflatable elements 1202 can be aligned along the lateral plane 502 that extends radially outward from the central axis 112. For example, the first location guide 302 can extend along the lateral plane 502 in a first direction from the central axis 112, and the second location guide 304 can extend in a second direction along the lateral plane 502 from the central axis 112. The inflatable elements 1202 may therefore be diametrically opposed to each other.

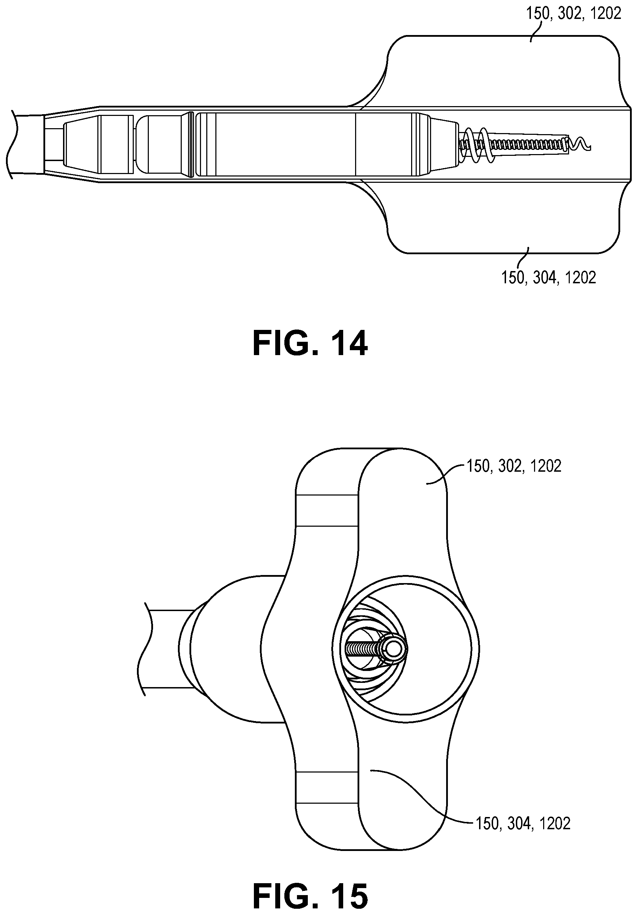

Referring to FIG. 14, a side view of a biostimulator system in a deployed state is shown in accordance with an embodiment. The inflatable elements 1202 can be inflated to the deployed state. For example, a saline solution or another inflation fluid may be introduced through inflation lumens that extend along a length of the biostimulator transport system 121 to the inflatable elements 1202. The inflation fluid can transfer into the inflatable elements 1202 to stretch the balloon material of the inflatable elements 1202. Inflatable elements 1202, which may be preformed balloons, for example, can inflate into a pre-shaped configuration. For example, the inflatable elements 1202 can expand into wings that extend radially outward from the central axis 112. The wings can have a higher profile in the deployed state than in the undeployed state.

Referring to FIG. 15, a perspective view of a biostimulator system in a deployed state is shown in accordance with an embodiment. The inflated wings can be taller in an axial direction than wide in a circumferential direction. The inflated location guides 150 can form a paddle extending along the lateral plane 502.

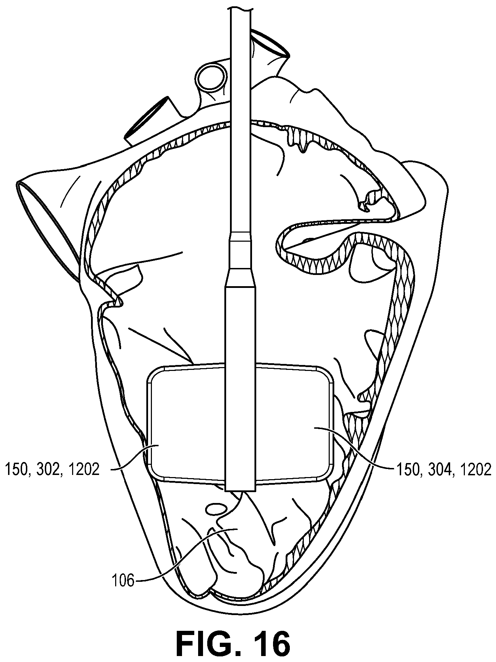

Referring to FIG. 16, a perspective view of a biostimulator system deployed in a target anatomy is shown in accordance with an embodiment. When expanded to the deployed state, the inflatable elements 1202 can engage the anatomical landmarks 152 of the heart 102. For example, inflatable elements 1202 can extend into the grooves of the ventricle. The paddle-shaped location guides 150 can fit in the grooves and orient the biostimulator transport system 121 in a predetermined orientation. Accordingly, the biostimulator 100 can be advanced from the lumen of the biostimulator transport system 121 toward the target tissue 106. After implantation, the inflatable elements 1202 can be deflated, and the biostimulator transport system 121 can be removed from the patient anatomy, leaving the biostimulator 100 implanted at the target tissue 106.

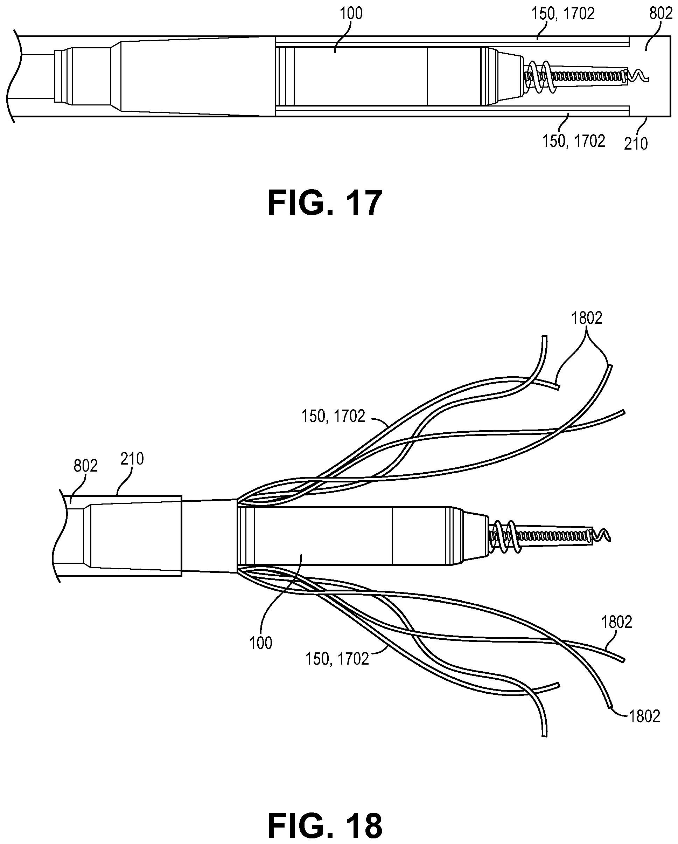

Referring to FIG. 17, a side view of a biostimulator system in an undeployed state is shown in accordance with an embodiment. The location guides 150 can provide visual feedback to a user, indicating an orientation of the biostimulator transport system 121 within the target anatomy. In an embodiment, the location guides 150 include radiopaque strands 1702. For example, the radiopaque strands 1702 may be wires or strands of radiopaque material, such as a flexible, radiopaque polymer wires, which extend distally from proximal ends to distal ends. The strands 1702 may be wholly or partially formed from the radiopaque material. For example, the strands 1702 can have non-radiopaque elongated bodies extending to radiopaque tips.

The proximal ends of the strands 1702 may, for example, be attached to catheter components of the biostimulator transport system 121 near the biostimulator coupling 122. For example, the strands 1702 may be attached to the catheter shaft or bearing housing of the biostimulator transport system 121. The biostimulator 100 may therefore move, e.g., spin, independently of the strands 1702. In an embodiment, the strands 1702 can be retracted into the outer sheath 210 along with the biostimulator 100. The strands 1702 can thereby be constrained in a low profile configuration in the undeployed state.

Referring to FIG. 18, a side view of a biostimulator system in a deployed state is shown in accordance with an embodiment. The outer sheath 210 can be retracted to expose the biostimulator 100 and the radiopaque strands 1702 to a surrounding environment. When exposed, the strands 1702 can extend radially outward to respective free ends 1802. For example, each strand 1702 can extend along a wavy path between a proximal end to a distal end. The proximal end of the strand 1702 can be nearer to the central axis 112 than the distal end, in the deployed state.

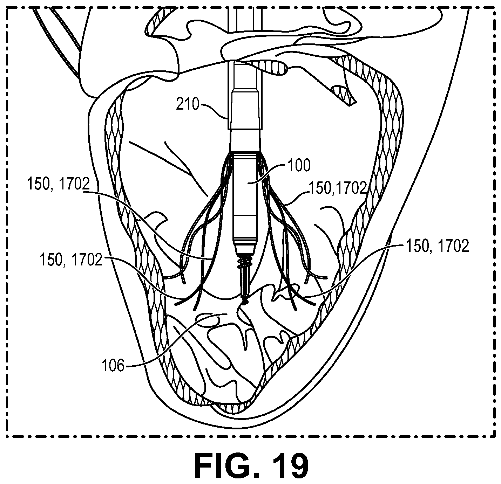

Referring to FIG. 19, a perspective view of a biostimulator system deployed in a target anatomy is shown in accordance with an embodiment. When the strands 1702 are expanded outward, the free ends 1802 can engage the anatomical landmarks 152 of the ventricle. For example, the strands 1702 can occupy the anterior and posterior grooves 152a, 152b of the ventricle. The strands 1702 may provide a visual aid to a user to understand how the biostimulator transport system 121 is oriented within the ventricle, and where the target tissue 106 is located. More particularly, a location of the grooves, or other heart structures, relative to the target tissue 106 can be determined. The user can therefore advance the biostimulator 100 from the outer sheath 210 toward the target tissue 106. The biostimulator 100 can be implanted at the target tissue 106, and the biostimulator transport system 121 may be detached from the biostimulator 100 and removed from the patient anatomy to complete the procedure.

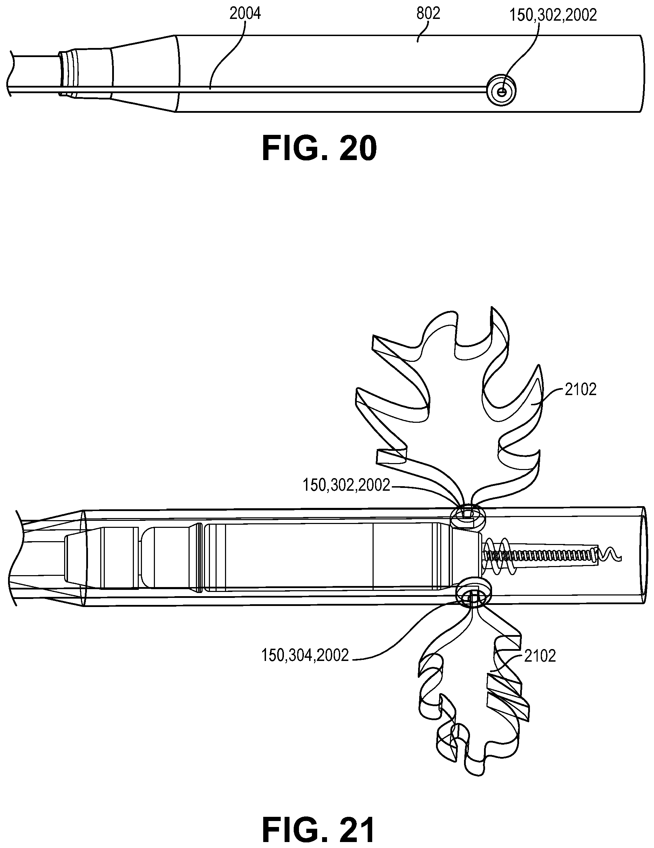

Referring to FIG. 20, a side view of a biostimulator system in an undeployed state is shown in accordance with an embodiment. Visual feedback may also be provided by contrast injections into the ventricle. In an embodiment, the location guides 150 include contrast ports 2002. For example, contrast ports 2002 may be located on the outer sheath 210 at diametrically opposed positions relative to the central axis 112. The contrast ports 2002 may be fed by injection lumens 2004 that extend along the catheter components of the biostimulator transport system 121.

Referring to FIG. 21, a side view of a biostimulator system in a deployed state is shown in accordance with an embodiment. The location guides 150 can provide visual feedback to a user regarding locations of the anatomical landmarks 152 by delivering contrast 2102 into the ventricle. For example, the contrast 2102 can be delivered radially outward through the contrast ports 2002 toward the anatomical landmarks 152. The user can view the dispersion of contrast 2102 under fluoroscopy to understand how the outer sheath 210 is oriented. For example, when the contrast 2102 spreads out within the grooves of the ventricle, the user can determine that the outer sheath 210 is oriented with the contrast ports 2002 in line with the grooves. When the contrast 2102 is not spread out, the user may determine that contrast ports 2002 are not aligned with the grooves.

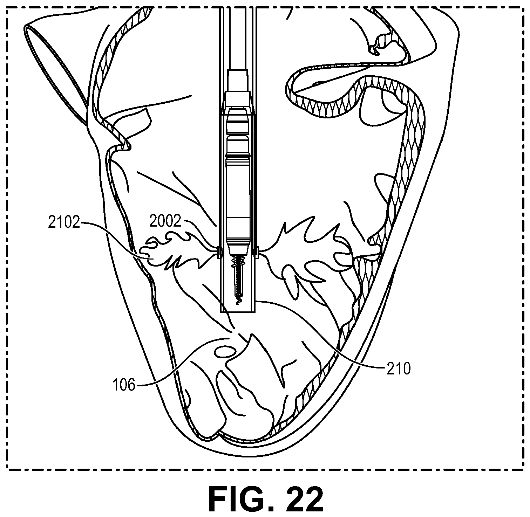

Referring to FIG. 22, a perspective view of a biostimulator system deployed in a target anatomy is shown in accordance with an embodiment. When the contrast 2102 is deployed through the ports 2306, the user can rotate the biostimulator transport system 121 until the visual feedback indicates that the ports 2306 are aligned with the grooves. The biostimulator 100 may then be advanced from the outer sheath 210 toward the target tissue 106. After implanting the biostimulator 100 in the target tissue 106, biostimulator 100 can be detached and the biostimulator transport system 121 can be removed from the patient anatomy.

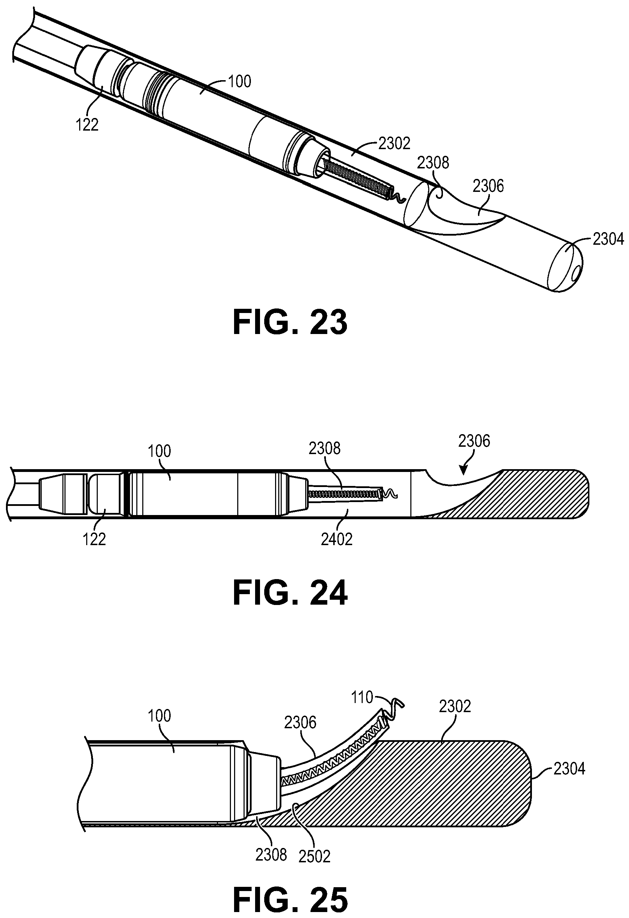

Referring to FIG. 23, a perspective view of a biostimulator system in an undeployed state is shown in accordance with an embodiment. The biostimulator transport system 121 can incorporate a protective sleeve 2302 that acts both as a guide and a deflection mechanism. More particularly, the protective sleeve 2302 may aid in implanting the biostimulator 100 by directing the biostimulator 100 toward the target tissue 106, e.g., to target the left bundle branch on the septal wall 104.

The protective sleeve 2302 may be formed from a soft polymer body having a solid, semirigid portion at a distal tip 2304. The protective sleeve 2302 can have a cylindrical outer wall extending to the distal tip 2304. In an embodiment, the protective sleeve 2302 includes a port 2306. The port 2306 can be an exit of a channel 2308 that extends along the central axis 112. The port 2306 can open laterally from the channel 2308. Accordingly, the biostimulator 100, which may sit within the channel 2308 in the undeployed state, may exit through the port 2306 toward a surrounding environment laterally around the protective sleeve 2302.

Referring to FIG. 24, a side view of a biostimulator system in an undeployed state is shown in accordance with an embodiment. The biostimulator 100 can be stored within the channel 2308 in the undeployed state. The biostimulator 100 can be connected to the biostimulator coupling 122, and movement of the biostimulator coupling 122 can cause the biostimulator 100 to advance or retract within the channel 2308.

Referring to FIG. 25, a side view of a biostimulator system in a deployed state is shown in accordance with an embodiment. The protective sleeve 2302 can include a ramp 2502 to transition from the channel 2308 to the port 2306. The ramp 2502 can act as a guide for the biostimulator 100. More particularly, when the biostimulator 100 is advanced through the channel 2308, it can ride over the ramp 2502 to achieve a sharp turn. The turn can direct a portion of the biostimulator 100, such as the fixation element 110 and/or electrode toward an optimal location at the target tissue 106, e.g., at or near a septal wall 104. The fixation element 110 of the biostimulator 100 can include in elongated nose, which is flexible and can bend laterally outward through the port 2306.

Steering of the nose of the biostimulator 100 can supplement, rather than replace, a steering capability of the catheter. It can be difficult to steer a catheter at a full orthogonal direction to the central axis 112. The ramp 2502, however, can help achieve such orthogonality by pushing and deflecting the flexible nose of the biostimulator 100 toward the target tissue 106.

In an embodiment, a distance between the distal tip 2304 of the protective sleeve 2302 and the port 2306 may be a predetermined distance. For example, the distance can equal the apex 152c of the ventricle and the target tissue 106. During device deployment, the biostimulator transport system 121 can be advanced until the distal tip 2304 is placed at the apex 152c. When so placed, the port 2306 can be at a height of the target tissue 106. The biostimulator transport system 121 can be rotated until the port 2306 is in line with the target tissue 106. The biostimulator 100 can then be advanced through the port 2306 to engage the target tissue 106 with the fixation element 110.

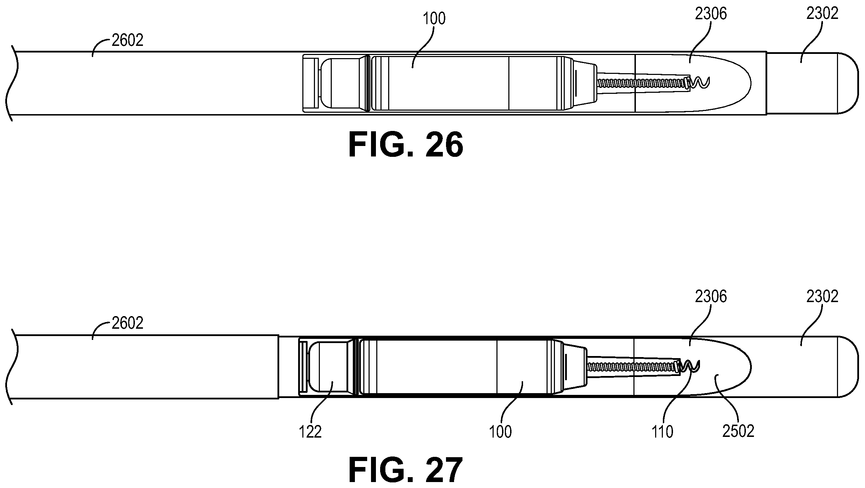

Referring to FIG. 26, a top view of a biostimulator system in an undeployed state is shown in accordance with an embodiment. In the undeployed state, the protective sleeve 2302 may be covered by an outer sleeve 2602. For example, the outer sleeve 2602 can be concentrically located with the protective sleeve 2302, and be an outermost structure of the sleeve assembly. The outer sleeve 2602 can be advanced to cover the port 2306. Accordingly, the biostimulator 100 can be constrained within the channel 2308 of the protective sleeve 2302.

Referring to FIG. 27, a top view of a biostimulator system in a deployed state is shown in accordance with an embodiment. To transition the biostimulator transport system 121 into the deployed state, the outer sleeve 2602 can be retracted over the protective sleeve 2302. The distal end of the outer sleeve 2602 can be pulled proximal to the port 2306. Accordingly, the ramp 2502 and/or the biostimulator 100 may be exposed to the surrounding environment. To implant the biostimulator 100, the biostimulator coupling 122 can be pushed forward to urge the biostimulator 100 forward along the ramp 2502. The nose of the biostimulator 100 can follow a curve of the channel 2308 and ramp 2502, and kick outward in the lateral direction. The fixation element 110 can contact the septal wall 104, and the biostimulator 100 may be rotated to implant at the target tissue 106. The biostimulator 100 can be detached, and the biostimulator transport system 121 may be removed from the patient anatomy.

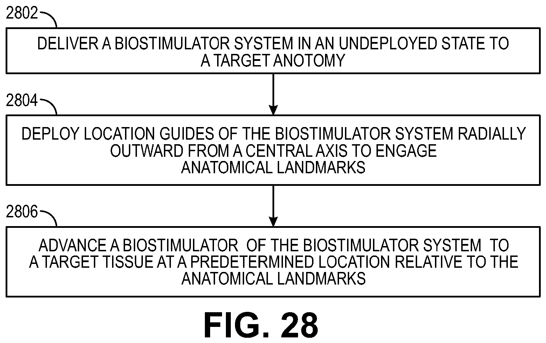

Referring to FIG. 28, a flowchart of a method of implanting a biostimulator in a target tissue is shown in accordance with an embodiment. At operation 2802, the biostimulator system 124 is delivered in the undeployed state to the target anatomy. For example, the biostimulator delivery system 124 can be introduced into the right ventricle of the heart 102. At operation 2804, the location guides 150 of the biostimulator system 124 are deployed. The location guides 150 can deploy radially outward from the central axis 112. For example, the location guides 150 can bow outward, splay radially, laterally, etc. The deployed location guides 150 engage the anatomical landmarks 152 in the target anatomy. Accordingly, an orientation of the biostimulator transport system 121, or a relative position between the target tissue 106 and the location guides 150, is known. At operation 2806, the biostimulator 100 is advanced to the target tissue 106. The biostimulator 100 can be introduced through the port 2306 of the biostimulator transport system 121 or otherwise steered to the target tissue 106. Steering can be made relative to the deployed location guides 150. More particularly, the target tissue 106 can be at the predetermined location relative to the anatomical landmarks 152, and the biostimulator 100 may be steered relative to those anatomical landmarks 152 to engage the target tissue 106. The biostimulator 100 can be detached from the biostimulator transport system 121 to remain intact and to pace the target tissue 106. The biostimulator transport system 121 may be removed from the patient anatomy.

In the foregoing specification, the invention has been described with reference to specific exemplary embodiments thereof. It will be evident that various modifications may be made thereto without departing from the scope of the invention as set forth in the following claims. The specification and drawings are, accordingly, to be regarded in an illustrative sense rather than a restrictive sense.

## Claims

1. A biostimulator transport system (121), comprising:
a biostimulator coupling (122) along a central axis (112), wherein the biostimulator coupling (122) is configured to hold a biostimulator (100); and
one or more location guides (150) deployable radially outward from the central axis (112) to engage anatomical landmarks (152), **characterized in that** the biostimulator coupling (122) is advanceable along the central axis (112) relative to the one or more location guides (150) when the one or more location guides (150) are in a deployed state.

2. The biostimulator transport system of claim 1, wherein the biostimulator transport system (121) comprises a plurality of location guides (150) including a first location guide (302) diametrically opposed to a second location guide (304).

3. The biostimulator transport system of claim 2, wherein the first location guide (302) and the second location guide (302) extend radially outward within a lateral plane (502).

4. The biostimulator transport system of claim 3, wherein the plurality of location guides (150) include a rear location guide (402) extending radially outward along a rear plane (602) orthogonal to the lateral plane (502).

5. The biostimulator transport system of any one of claims 1 to 4, wherein the biostimulator transport system (121) comprises a plurality of location guides (150) including a plurality of wires (902) deployable from an undeployed state to a deployed state.

6. The biostimulator transport system of claim 5, wherein in the undeployed state the plurality of wires (902) extends longitudinally parallel to the central axis (112), and wherein in the deployed state the plurality of wires (902) bow radially outward.

7. The biostimulator transport system of claim 5 or 6, further comprising a skin (1002) covering the plurality of wires (902).

8. The biostimulator transport system of claim 7, wherein the skin (1002) deforms radially outward when the plurality of wires (902) are deployed.

9. The biostimulator transport system of any one of claims 1 to 8, wherein the biostimulator transport system (121) comprises a plurality of location guides (150) deploying asymmetrically relative to the central axis (112).

10. The biostimulator transport system of any one of claims 1 to 9, wherein the one or more location guides (150) extend from respective proximal ends proximal to the biostimulator coupling (122).

11. The biostimulator transport system of claim 1, wherein the biostimulator transport system (121) comprises a plurality of location guides (150) including a plurality of inflatable elements (1202) deployable from an undeployed state to a deployed state.

12. The biostimulator transport system of claim 11, wherein in the undeployed state the plurality of inflatable elements (1202) is deflated, and wherein in the deployed state the plurality of inflatable elements (1202) is inflated.

13. The biostimulator transport system of claim 1, wherein the biostimulator transport system (121) comprises a plurality of location guides (150) including a plurality of radiopaque strands (1702) extending radially outward to respective free ends (1802).

14. The biostimulator transport system of claim 1, wherein the biostimulator transport system (121) comprises a plurality of location guides (150) including a plurality of contrast ports (2002) to deliver contrast radially outward toward the anatomical landmarks (152).

15. A biostimulator system (124), comprising:
the biostimulator transport system (121) of any of claims 1-14; and
a biostimulator (100) mounted on the biostimulator coupling (122), wherein the biostimulator (100) includes a body (103) having an electronics compartment (120) containing circuitry.

## Patentansprüche

1. Biostimulatortransportsystem (121), umfassend:
eine Biostimulatorkupplung (122) entlang einer Mittelachse (112), wobei die Biostimulatorkupplung (122) dazu konfiguriert ist, einen Biostimulator (100) zu halten; und
eine oder mehrere Positionsführungen (150), die von der Mittelachse (112) radial nach außen einsetzbar sind, um anatomische Landmarken (152) in Eingriff zu nehmen, **dadurch gekennzeichnet, dass** die Biostimulatorkupplung (122) entlang der Mittelachse (112) relativ zu der einen oder den mehreren Positionsführungen (150) vorschiebbar ist, wenn sich die eine oder die mehreren Positionsführungen (150) in einem eingesetzten Zustand befinden.

2. Biostimulatortransportsystem nach Anspruch 1, wobei das Biostimulatortransportsystem (121) eine Vielzahl von Positionsführungen (150) umfasst, die eine erste Positionsführung (302) beinhaltet, die einer zweiten Positionsführung (304) diametral gegenüberliegt.

3. Biostimulatortransportsystem nach Anspruch 2, wobei sich die erste Positionsführung (302) und die zweite Positionsführung (302) innerhalb einer seitlichen Ebene (502) radial nach außen erstrecken.

4. Biostimulatortransportsystem nach Anspruch 3, wobei die Vielzahl von Positionsführungen (150) eine hintere Positionsführung (402) beinhaltet, die sich entlang einer hinteren Ebene (602), die orthogonal zu der seitlichen Ebene (502) verläuft, radial nach außen erstreckt.

5. Biostimulatortransportsystem nach einem der Ansprüche 1 bis 4, wobei das Biostimulatortransportsystem (121) eine Vielzahl von Positionsführungen (150) umfasst, die eine Vielzahl von Drähten (902) beinhaltet, die von einem nicht eingesetzten Zustand in einen eingesetzten Zustand einsetzbar sind.

6. Biostimulatortransportsystem nach Anspruch 5, wobei sich die Vielzahl von Drähten (902) in dem nicht eingesetzten Zustand in Längsrichtung parallel zu der Mittelachse (112) erstreckt und wobei sich die Vielzahl von Drähten (902) in dem eingesetzten Zustand radial nach außen biegt.

7. Biostimulatortransportsystem nach Anspruch 5 oder 6, ferner umfassend eine Haut (1002), welche die Vielzahl von Drähten (902) abdeckt.

8. Biostimulatortransportsystem nach Anspruch 7, wobei sich die Haut (1002) radial nach außen verformt, wenn die Vielzahl von Drähten (902) eingesetzt wird.

9. Biostimulatortransportsystem nach einem der Ansprüche 1 bis 8, wobei das Biostimulatortransportsystem (121) eine Vielzahl von Positionsführungen (150) umfasst, die asymmetrisch relativ zu der Mittelachse (112) eingesetzt sind.

10. Biostimulatortransportsystem nach einem der Ansprüche 1 bis 9, wobei sich die eine oder die mehreren Positionsführungen (150) aus jeweiligen proximalen Enden proximal zu der Biostimulatorkupplung (122) erstrecken.

11. Biostimulatortransportsystem nach Anspruch 1, wobei das Biostimulatortransportsystem (121) eine Vielzahl von Positionsführungen (150) umfasst, die eine Vielzahl von aufblasbaren Elementen (1202) beinhaltet, die von einem nicht eingesetzten Zustand in einen eingesetzten Zustand einsetzbar ist.

12. Biostimulatortransportsystem nach Anspruch 11, wobei die Vielzahl von aufblasbaren Elementen (1202) in dem nicht eingesetzten Zustand entleert ist und wobei die Vielzahl von aufblasbaren Elementen (1202) in dem eingesetzten Zustand aufgeblasen ist.

13. Biostimulatortransportsystem nach Anspruch 1, wobei das Biostimulatortransportsystem (121) eine Vielzahl von Positionsführungen (150) umfasst, die eine Vielzahl von röntgendichten Strängen (1702) beinhaltet, die sich zu jeweiligen freien Enden (1802) radial nach außen erstrecken.

14. Biostimulatortransportsystem nach Anspruch 1, wobei das Biostimulatortransportsystem (121) eine Vielzahl von Positionsführungen (150) umfasst, die eine Vielzahl von Kontrastanschlüssen (2002) beinhaltet, um Kontrast in Richtung der anatomischen Landmarken (152) radial nach außen abzugeben.

15. Biostimulatorsystem (124), umfassend:
das Biostimulatortransportsystem (121) nach einem der Ansprüche 1-14; und
einen Biostimulator (100), der auf der Biostimulatorkupplung (122) montiert ist, wobei der Biostimulator (100) einen Körper (103) beinhaltet, der ein Elektronikfach (120) aufweist, das eine Schaltung enthält.

## Revendications

1. Système de transport de biostimulateur (121), comprenant :
un raccord de biostimulateur (122) le long d'un axe central (112), dans lequel le raccord de biostimulateur (122) est configuré pour maintenir un biostimulateur (100) ; et
au moins un guide de localisation (150) déployable radialement vers l'extérieur depuis l'axe central (112) pour venir au contact de repères anatomiques (152), **caractérisé en ce que** le raccord de biostimulateur (122) peut être avancé le long de l'axe central (112) par rapport audit au moins un guide de localisation (150) lorsque ledit au moins un guide de localisation (150) se trouve dans un état déployé.

2. Système de transport de biostimulateur selon la revendication 1, dans lequel le système de transport de biostimulateur (121) comprend une pluralité de guides de localisation (150) incluant un premier guide de localisation (302) diamétralement opposé à un second guide de localisation (304).

3. Système de transport de biostimulateur selon la revendication 2, dans lequel le premier guide de localisation (302) et le second guide de localisation (302) s'étendent radialement vers l'extérieur dans un plan latéral (502).

4. Système de transport de biostimulateur selon la revendication 3, dans lequel la pluralité de guides de localisation (150) inclut un guide de localisation arrière (402) s'étendant radialement vers l'extérieur le long d'un plan arrière (602) orthogonal au plan latéral (502).

5. Système de transport de biostimulateur selon l'une quelconque des revendications 1 à 4, dans lequel le système de transport de biostimulateur (121) comprend une pluralité de guides de localisation (150) incluant une pluralité de fils (902) déployables d'un état non déployé à un état déployé.

6. Système de transport de biostimulateur selon la revendication 5, dans lequel, dans l'état non déployé, la pluralité de fils (902) s'étend longitudinalement parallèlement à l'axe central (112), et dans lequel, dans l'état déployé, la pluralité de fils (902) se courbent radialement vers l'extérieur.

7. Système de transport de biostimulateur selon la revendication 5 ou 6, comprenant en outre une membrane (1002) recouvrant la pluralité de fils (902).

8. Système de transport de biostimulateur selon la revendication 7, dans lequel la membrane (1002) se déforme radialement vers l'extérieur lorsque la pluralité de fils (902) est déployée.

9. Système de transport de biostimulateur selon l'une quelconque des revendications 1 à 8, dans lequel le système de transport de biostimulateur (121) comprend une pluralité de guides de localisation (150) se déployant de manière asymétrique par rapport à l'axe central (112).

10. Système de transport de biostimulateur selon l'une quelconque des revendications 1 à 9, dans lequel ledit au moins un guide de localisation (150) s'étend depuis des extrémités proximales respectives situées de façon proximale par rapport au raccord de biostimulateur (122).

11. Système de transport de biostimulateur selon la revendication 1, dans lequel le système de transport de biostimulateur (121) comprend une pluralité de guides de localisation (150) incluant une pluralité d'éléments gonflables (1202) déployables d'un état non déployé à un état déployé.

12. Système de transport de biostimulateur selon la revendication 11, dans lequel, dans l'état non déployé, la pluralité d'éléments gonflables (1202) est à l'état dégonflé, et dans lequel, dans l'état déployé, la pluralité d'éléments gonflables (1202) est à l'état gonflé.

13. Système de transport de biostimulateur selon la revendication 1, dans lequel le système de transport de biostimulateur (121) comprend une pluralité de guides de localisation (150) incluant une pluralité de brins radio-opaques (1702) s'étendant radialement vers l'extérieur jusqu'à des extrémités libres respectives (1802).

14. Système de transport de biostimulateur selon la revendication 1, dans lequel le système de transport de biostimulateur (121) comprend une pluralité de guides de localisation (150) incluant une pluralité d'orifices d'injection de contraste (2002) destinés à délivrer un produit de contraste radialement vers l'extérieur vers les repères anatomiques (152).

15. Système de biostimulateur (124), comprenant :
le système de transport de biostimulateur (121) selon l'une quelconque des revendications 1 à 14 ; et
un biostimulateur (100) monté sur le raccord de biostimulateur (122), dans lequel le biostimulateur (100) comprend un corps (103) présentant un compartiment électronique (120) contenant des circuits.
